Europäisches Patentamt

European Patent Office

Office européen des brevets

(11) Publication number: **0 072 925**
**B1**

# (12) EUROPEAN PATENT SPECIFICATION

(45) Date of publication of patent specification: 25.05.88

(21) Application number: 82106599.2

(22) Date of filing: 21.07.82

(51) Int. Cl.⁴: **C 12 N 15/00,** C 07 H 21/04, **C 12 N 1/20**

(54) T4 DNA fragment as a stabilizer for proteins expressed by cloned DNA.

(30) Priority: 17.08.81 US 293614

(43) Date of publication of application:
02.03.83 Bulletin 83/09

(45) Publication of the grant of the patent:
25.05.88 Bulletin 88/21

(84) Designated Contracting States:
AT BE CH DE FR GB IT LI LU NL SE

(56) References cited:
CHEMICAL ABSTRACTS, vol. 94, no. 25, 22nd
June 1981, page 285, no. 205151n, Columbus,
Ohio, USA

CHEMICAL ABSTRACTS, vol. 90, no. 23, 4th
June 1979, page 311, no. 182893g, Columbus,
Ohio, USA

CHEMICAL ABSTRACTS, vol. 75, no. 17, 25th
October 1971, page 120, no. 106460p,
Columbus, Ohio, USA

LEE D. SIMON et al.: "Bacteriophages inhibit
degradation of abnormal proteins in E. coli" &
DHEW PUBL. (NIH) (U.S.) 1979, NIH-79-1591,
LTD. PROTEOLYSIS MICROORG., 115-121
LEE D. SIMON et al.: "Bacteriophages inhibit
degradation of abnormal proteins in E. coli" &

(73) Proprietor: Rutgers Research and Educational
Foundation
New Brunswick New Jersey (US)

(72) Inventor: Simon, Lee D.
311 South 41 Street
Philadephia, Pa. 19104 (US)
Inventor: Fay, Rose B.
98A Rutgers Road
Piscataway New Jersey 08854 (US)

(74) Representative: Vossius & Partner
Siebertstrasse 4 P.O. Box 86 07 67
D-8000 München 86 (DE)

(56) References cited:
NATURE (LONDON) 1978, 275(5679), 424-428
ROBERT L. SWIFT et al.: "Bacteriophage T4
inhibits colicin E2-induced degradation of
Escherichia coli deoxyribonucleicacid. I.
Protein synthesis-dependent inhibition" &
J.VIROL. 1971, 8(3), 303-310

Courier Press, Leamington Spa, England.

# 0 072 925

**Description**

Background of the invention

This invention relates to the field of recombinant DNA. In one of its aspects, this invention relates to a DNA fragment which inhibits degradation of abnormal proteins in bacterial cells. In another aspect, this invention relates to a method of isolating this stabilizing DNA fragment from a T-even phage particles. In another aspect, this invention relates to a plasmid containing the stabilizing DNA fragment. In yet another aspect, this invention relates to a method of amplifying foreign proteins encoded by cloned DNA by including the stabilizing DNA fragment into the genetic machinery of bacterial host cells.

Most living cells possess efficient systems for recognizing the eliminating abnormal proteins. As used herein, the term "abnormal proteins" refers to proteins with abnormal conformations, protein fragments, polypeptide sequences containing amino acid analogues, missense mutant proteins, nonsense protein fragments, proteins encoded by cloned DNA and other polypeptides not ordinarily present in healthy, viable cells. In *E. coli* cells, for example, the half-lives of protein fragments and protein with abnormal conformations are much shorter than the half-lives of normal proteins. About 3% of normal proteins turns over each hour in *E. coli* cells. Abnormal proteins, however, are so short-lived that they are found in much lower quantities than normal proteins or fail to accumulate to detectable levels.

Little is known about the mechanism by which living cells detect and degrade abnormal proteins. Some information is available about this system in *E. coli* cells. See, for example, Simon et al., *Nature, 275*, 424 (1978). It is known that the degradation of abnormal proteins in *E. coli* cells is ATP-dependent. If cellular ATP levels are substantially reduced, the turnover of abnormal proteins and protein fragments is also reduced. It is also believed that the ATP-dependent degradative mechanism is bound to the *E. coli* cell membrane. However, the identities and functions of the enzymes responsible for recognizing and degrading abnormal proteins remain obscure in *E. coli* and in other bacterial cells.

The degradation systems possessed by living cells present a major obstacle to the manufacture of useful proteins by means of recombinant DNA or molecular cloning procedures. By these procedures, the genes which code for eukaryotic proteins are introduced into host cells, such as bacterial cells, which then express the foreign genes as the eukaryotic proteins. The recombinant DNA procedures involve isolating the relevant genes, inserting them into suitable cloning vehicles, such as bacterial plasmids or viruses, and transforming host cells by introducing the hybrid cloning vehicles into the cells. The transformed cells which contain the relevant genes are then selected from all the other cells and are grown in cultures. By such techniques, bacterial cells have been induced thus far to manufacture human growth hormone, human insulin, human interferon, etc. However, the bacterial cells apparently recognize these products as abnormal proteins, and under most circumstances, degrade them. Thus, the ability of living cells to detect and degrade abnormal proteins may severely limit the yields of useful proteins obtained from cloned cells.

To increase such yields, it would be highly desirable to provide a method for inhibiting or suppressing the degradation mechanism in cells carrying cloned genes. Such a method, when combined with already known recombinant DNA methods, would provide a highly efficient means for producing useful proteins.

It has previously been reported that the bacteriophage known as T4 influences the degradation function in *E. coli* cells. Simon et al., in *Nature, supra*, reported that T4 infection of *E. coli* cells inhibits degradation of abnormal polypeptide sequences but does not affect the turnover rate of normal bacterial proteins. In the studies reported therein, it was found that adsorption of T4 particles to the *E. coli* surface and injection of T4 DNA were not by themselves sufficient to alter protein turnover. It was found that inhibition of degradation requires the synthesis of early T4 proteins. It was also reported therein that other phages, such as T5 and T7, also inhibit degradation in *E. coli* cells but not so to great an extent as does T4 phage. However, infecting *E. coli* cells with T4, T5 or T7, phage particles is fatal to the host cells, and this method is unsuitable for amplifying proteins expressed by cloned DNA.

The bacteriophage T4 together with the bacteriophages T2 and T6 form a family of bacteriophages known as T-even phages. The T-even phages are almost identical in structure, composition and properties. Thus, one of ordinary skill in the art would conclude that all members of the T-even bacteriophage family contain genes which suppress degradation.

In order to maximize the yields of eukaryotic proteins produced by recombinant DNA techniques, it would be highly desirable to excise from T-even phages, especially T4 phage particles the gene(s) responsible for inhibiting the degradation mechanism and to insert the same gene(s) into bacterial host cells. In this manner, the degradation mechanism of cells carrying cloned DNA can be turned off without killing the cells as by infection with T4 phage particles.

It is an object of the invention to provide a DNA fragment from T-even phage particles which will inhibit the degradation of abnormal proteins in bacterial host cells.

It is another object of the invention to provide a cloning vehicle such as a bacterial plasmid, which contains the stabilizing T-even DNA fragment.

It is another object of the invention to provide bacterial cells containing the hybrid cloning vehicle with the stabilizing T-even DNA fragment.

It is still another object of the invention to provide transformants which contain the genes for producing eukaryotic proteins as well as the stabilizing T4 DNA fragment.

2

# 0 072 925

It is still another object of the invention to provide a method for suppressing the degradation of abnormal proteins in a bacterial cell.

It is still another object of the invention to provide a DNA fragment coding for protein that suppresses degradation of abnormal proteins in bacterial cells.

These objects are accomplished by means of the present invention.

Summary of the invention

A primary subject matter of the invention is a DNA fragment isolated from the DNA of a T-even bacteriophage, said DNA fragment consisting essentially of a sequence of nucleotide bases which code for protein that suppresses degradation of abnormal polypeptides in bacterial cells.

The said DNA fragment may be a fragment wherein the nucleotide bases code for protein that suppresses degradation in gram-negative bacterial cells.

Preferably the said DNA fragment is a fragment wherein the nucleotide bases code for protein that suppresses degradation in *E. coli* cells.

The said DNA fragment may also be a fragment wherein the nucleotide bases code for protein that suppresses degradation in *Pseudomonas, Aerobacter, Salmonella, Proteus* or *Serratia* cells.

The said DNA fragment can further be a fragment wherein the nucleotide bases code for protein that suppresses degradation in gram-positive bacterial cells.

Preferably the latter DNA fragment is a fragment wherein the nucleotide bases code for protein that suppresses degradation in *Bacillus subtilis* cells.

Preferably, the said DNA fragment is isolated from T4 bacteriophage. This DNA fragment preferably comes from the vicinity of gene 49 of the T4 genetic map.

The T-even DNA from which the DNA fragment of the invention stems is preferably cytosine-containing and nonglucosylated.

Another subject matter of the invention is a hybrid cloning vehicle comprising a cloning vehicle and the DNA fragment of the invention.

Said cloning vehicle of the invention is preferably a plasmid, the plasmid pBR 325 being especially preferred.

In another aspect of the invention the hybrid cloning vehicle further comprises a sequence of nucleotide bases coding for a eukaryotic, preferably human, protein.

The said human protein is preferably human interferon.

Another subject matter of the invention is a bacterial cell containing the hybrid cloning vehicle of the invention.

Preferred bacterial cells containing the hybrid cloning vehicles are *E. coli* cells, *Bacillus subtilis* cells, *Pseudomonas, Aerobacter, Salmonella, Proteins* and *Serratia* cells.

The said bacterial cells containing the hybrid cloning vehicle of the invention may further contain a second cloning vehicle, said second cloning vehicle containing a sequence of nucleotide bases coding for a eukaryotic protein.

Again *E. coli* cells, *Bacillus subtilis* cells, *Pseudomonas, Aerobacter, Salmonella, Proteus* and *Serratia* cells are preferred.

A further subject matter of the invention is a method for suppressing the degradation of abnormal proteins in a bacterial cell, comprising producing the DNA fragment of the invention, inserting said DNA fragment into a cloning vehicle to produce a hybrid cloning vehicle, and transforming a bacterial cell by introducing said hybrid cloning vehicle therein.

In the said method of the invention the cloning vehicle is preferably a plasmid and the bacterial cells are *E. coli* cells, *Bacillus subtilis* cells, *Pseudomonas, Aerobacter, Salmonella, Proteus* or *Serratia* cells.

The T-even DNA from which the DNA fragment used in the method of the invention stems is preferably cytosine-containing and nonglucosylated. The DNA fragment used is preferably isolated from T4 bacteriophage.

Another subject matter of the invention is a method for producing a DNA fragment consisting essentially of a sequence of nucleotide bases coding for protein that suppresses degradation of abnormal proteins in bacterial cells, comprising treating the DNA of a T-even bacteriophage with a restriction enzyme to cleave the DNA into fragments, at least one of the said fragments coding for protein that suppresses degradation in bacterial cells, inserting said DNA fragments into cloning vehicles to produce hybrid cloning vehicles, transforming bacterial cells by introducing said hybrid cloning vehicles therein, and selecting the transformants exhibiting suppressed degradation of abnormal proteins.

In this method of the invention the cloning vehicle is again preferably a plasmid and the bacterial cells are *E. coli* cells, *Bacillus subtilis* cells, *Pseudomonas, Aerobacter, Salmonella, Proteus* or *Serratia* cells.

The T-even DNA from which the DNA fragment used in this method of the invention stems is preferably cytosine-containing containing and nonglucosylated. The DNA fragment used is preferably isolated from T4 bacteriophage.

The said method of the invention preferably further comprises removing said hybrid cloning vehicle from a selected transformant and excising said DNA fragment from said hybrid cloning vehicle.

A further subject matter of the invention is a nucleic acid consisting essentially of a sequence of nucleotide bases coding for protein that suppresses degradation of abnormal proteins in bacterial cells.

3

**0 072 925**

The said nucleic acid is a DNA fragment which preferably codes for protein that suppresses degradation in *E. coli* cells, or in *Bacillus subtilis* or in the other microorganisms mentioned above.

A further subject matter of the invention is a hybrid cloning vehicle comprising a cloning vehicle and the said DNA fragment.

Said cloning vehicle is preferably a plasmid, the plasmid pBR 325 being especially preferred.

Preferably said cloning vehicle further comprises a sequence of nucleotide bases coding for a eukaryotic protein.

A further subject matter of the invention is a bacterial cell containing the said hybrid cloning vehicle.

The said bacterial cell is preferably an *E. coli* cell or a *Bacillus subtilis* cell or one of the other microorganisms mentioned above.

Another subject matter of the invention finally is a method for suppressing the degradation of abnormal proteins in a bacterial cell comprising producing the above-mentioned DNA fragment, inserting said DNA fragment into a cloning vehicle to produce a hybrid cloning vehicle, and transforming a bacterial cell by introducing said hybrid cloning vehicle therein.

In this method of the invention the cloning vehicle used is preferably a plasmid and the bacterial cell is an *E. coli* cell or a *Bacillus subtilis* cell or one of the other microorganisms mentioned above.

The method of isolating the stabilizing DNA fragment according to the present invention comprises treating the DNA of a T-even bacteriophage with a restriction enzyme to cleave the DNA into fragments, at least one of said fragments coding for protein that causes the suppression of degradation of abnormal proteins in bacterial cells, inserting said DNA fragments into cloning vehicles, such as bacterial plasmids, to produce hybrid cloning vehicles, transforming bacterial cells by introducing said hybrid cloning vehicles therein, and selecting the transformants which exhibit suppressed degradation of abnormal proteins. As used herein, the term "hybrid cloning vehicle" refers broadly to a cloning vehicle, such as a bacterial plasmid, containing a T-even DNA insert.

The method of the invention leads to an increase in the yield of proteins expressed by cloned DNA in a bacterial cell by inserting the DNA fragment into a cloning vehicle, such as a bacterial plasmid, to produce a hybrid cloning vehicle, and transforming the bacterial cell containing the cloned DNA by introducing said hybrid cloning vehicle into said bacterial cell.

Detailed description of the invention

The invention will now be explained in more detail with reference being made to T4 phage which is preferred for the isolation of the DNA fragment of the invention. It is expressly stated however, that the invention is not restricted to this embodiment, but that other T-even phages can also be used.

The genetic map of T4 phage is known to be circular and to contain about 150 genes. About 40 different T4 gene products, each coded by a specific T4 gene, interact to produce the coat proteins which form the mature virus particle. It is not known how many genes are responsible for suppressing degradation of abnormal proteins in bacterial hosts. Nor is it known which proteins are expressed by these genes. However, as a result of the practices of the present invention, it has been learned that a DNA fragment which codes for protein that functions to suppress degradation of abnormal proteins in bacterial cells originates from the vicinity of gene 49 of the T4 genetic map.

Wais, et. al., in *Virology*, *39*, 153 (1969), have reported that T4 phage is able to replicate when injected, into a variety of bacteria, including *E. coli* strain B/4, *Salmonella*, *Aerobacter*, *Proteus*, and *Serratia*. Thus, the genetic machinery of T4 is unimpaired and is able to function essentially normally when inserted in a wide variety of host cells.

The genetic nucleic acid present in the core of the T4 phage particle is double-stranded DNA. T4 DNA comprises about $3.6 \times 10^5$ base pairs (MW ca. $1.2 \times 10^8$). There are twice as many A—T base pairs as G—C base pairs. T4 DNA is unusual in that it contains the base 5—OH— methylcytosine in place of the more usual cytosine. Moreover, one or more glucose residues are attached to some of the 5—$CH_2OH$ cytosine groups in the T4 DNA. 5-OH-methylcyrosine, like cytosine, forms base pairs with guanine. The presence of 5-OH-methylcytosine and its glucose derivatives in place of cytosine has no bearing on the genetic properties of T4 phage. The biological significance of the unusual base has not yet been clearly established although it is believed that its function is to protect T4 DNA from a phage specific enzyme which destroys unmodified DNA.

In accordance with the present invention, a process is disclosed for isolating a specific DNA fragment from T-even phage particles which will suppress degradation of abnormal proteins, such as foreign proteins encoded by cloned DNA, in bacterial cells. The process begins with the extraction of DNA from the capsids of T-even phage particles.

T-even DNA may be extracted from the capsids of the T-even particles by well known methods. Both chemical or mechanical methods are known to remove the DNA from the T-even particles although chemical methods are preferred since they are less likely to damage the DNA. For purposes of the present invention, it is preferable that the DNA come from T-even mutants in which the DNA is cytosine-containing and nonglucosylated. This form of DNA is preferred since most restriction enzymes will not act on wild-type T4 DNA. Furthermore, wild-type T4 DNA may not be transcribed in host cells as well as cytosine-containing, nonglucosylated DNA.

After the DNA has been extracted from the T-even phage particles, the DNA is digested with a suitable

4

restriction endonuclease. The restriction enzyme scans the DNA strand and cleaves it into fragments whenever a particular short sequence of nucleotides is encountered. Each restriction enzyme therefore cleaves the T-even DNA into a characteristic set of fragments which can be separated, if desired, by well known methods such as gel electrophoresis. It is essential that the restriction enzyme not cleave the DNA within the gene(s) responsible for suppressing degradation of abnormal proteins. A variety of restriction enzymes have been found suitable for the practice of the present invention, including such well known endonucleases as Pst I and Eco RI.

To isolate the specific T-even DNA fragment containing the stabilizing gene(s), the fragments are inserted into suitable cloning vehicles to produce hybrid cloning vehicles. Both viruses and plasmids are suitable cloning vehicles; however, bacterial plasmids carrying genes which serve as recognition markers, such as antibiotic resistance, may be preferred. For example, the readily available plasmid pBR 325, which confers resistance to amplicillin, chloremphenicol, and tetracycline, or the readily available plasmid pBR 322, which confers resistance to ampicillin and tetracycline, are suitable cloning vehicles for the present invention.

The T-even DNA fragments can be inserted into the cloning vehicles, such as the bacterial plasmids, by a variety of enzymatic techniques. When the T-even DNA has been cleaved with a restriction enzyme leaving "sticky ends", the same restriction enzyme can be used to cleave open the appropriate bacterial plasmid. The bacterial plasmid will then have matching sticky ends and the DNA fragments can be directly annealed to the bacterial plasmids. However, if the restriction enzyme leaves blunt ends, the enzyme terminal transferase can be used to provide a short sequence of identical bases, such as four cytosines, to the DNA fragments. The fragments can then be annealed to the plasmid DNA to which a complementary sequence of bases (four guanines) have been added.

The hybrid cloning vehicles are next inserted into bacterial host cells. Treatment with a dilute solution of calcium chloride is known to render the cell walls permeable to plasmids. *E. coli* cells have been found to be suitable host cells for expressing the stabilizing DNA fragment although the method of the present invention is not confined to these cells. *E. coli* is classified as a gram-negative species of bacteria. Other gram-negative bacteria such as *Pseudomonas, Aerobacter,* etc., are also suitable hosts for expressing the stabilizing function of the DNA fragment. With suitable modification in the Shine-Dalgar sequence, gram-positive bacteria, such as *Bacillus subtilis*, will also serve as suitable hosts for the stabilizing DNA fragment. The modification techniques are conventionally known in the art.

To select the host cells which have been transformed by the hybrid cloning vehicles, bacteria are grown first in a non-selective medium and then in a selective medium. For example, the restriction enzyme Pst I cuts the plasmid of pBR 325 in the midst of the gene which confers ampicillin resistance, but leaves the genes which confer chloramphenicol and tetracycline resistance intact. Thus, one can select the transformed cells containing inserts in the Pst I site of pBR 325 by screening for resistance to chloramphenicol and tetracycline and sensitivity to ampicillin.

To isolate those transformants containing the stabilizing DNA fragment, the transformants are screened for those which exhibit suppressed degradation of abnormal proteins. A test based upon the ability of temperature sensitive phage mutants to propagate in the transformants has been found suitable for this purpose. At appropriate temperatures, the phage mutant will only grow in host cells having defective mechanisms for degradation of abnormal proteins. For example, the phage mutant known as λ0ts is a λ phage with a defect in gene *0*. The λ0 protein is vital to the survival of the virus. Wild-type phage is able to propagate in *E. coli* at 30° and at 39°C. λ0ts, however, is unable to propagate at 39°C unless the degradation mechanism of the *E. coli* cells is impaired (see Simon et al., *Proc. Nat. Acad. Sci. USA, 76*, 1623 (1979)). Thus, the transformants containing the desired T-even DNA fragment can be selected by their ability to propagate the λ0ts phage mutants at 30°C and at 39°C. If desired, these transformants can be tested further for their ability to degrade abnormal proteins and protein fragments by methods described in Simon et al., *Nature, supra.*

In this manner transformants are isolated having an impaired degradation mechanism. The transformants contain a hybrid cloning vehicle such as a plasmid, in which a DNA fragment from T-even phage has been inserted. This DNA fragment is functional in bacteria, and contains the genetic information required to inhibit abnormal protein degradation.

The plasmid can be removed from the selected transformants and introduced into other host cells where it will inhibit degradation in the new host cells. The new host cells are not confined to *E. coli* but may be any of a wide variety of bacterial cells, including *Pseudomonas, Aerobacter*, etc., and, with suitable modification of the Shine-Dalgar sequence, *Bacillus subtilis*, etc. The new host cells may additionally carry an appropriate recombinant plasmid for producing eukaryotic proteins. The presence of the stabilizing DNA fragment will increase dramatically the yields of the eukaryotic proteins.

Alternatively, the T-even DNA fragment may be excised from the hybrid plasmid by treatment with a restriction enzyme, for example, Pst I. The DNA fragment may then be inserted into a cloning vehicle, such as a plasmid, already carrying a gene for producing a eukaryotic protein. When the new recombinant plasmid is introduced into a host cell, the yields of the eukaryotic protein will be greatly increased.

After the stabilizing DNA fragment has been isolated, it may be subjected to further analysis and study. For example, the DNA fragment can be sequenced by laborious but well known techniques. The DNA fragment may also be subcloned to find the smallest DNA fragment which would inhibit degradation in

**0 072 925**

bacterial cells. Subcloning can be accomplished by digesting the stabilizing DNA fragment with a variety of restriction enzymes in order to cut it into smaller fragments. Each of these pieces can then be inserted into a plasmid and then cloned in a bacterial host. The hosts can then be screened for impaired degradation. In this manner, the smallest DNA fragment can be isolated which codes for protein that suppresses degradation of abnormal proteins in bacteria.

The practices of the present invention may be further illustrated by the following examples.

Example 1

For purposes of testing the practices of the present invention, DNA was extracted by well known methods from T4 mutants having the following mutations: alc⁻, den A⁻, *den B⁻*, 42⁻, 56⁻. These mutations result in T4 particles having cytosine-containing, nonglycosylated DNA.

The DNA from the T4 mutants was cleaved with the restriction endonuclease Pst I. This restriction enzyme recognizes the nucleotide sequence CTGCA $\downarrow$ G and cuts the T4 DNA between A and G wherever this sequence appears. Digestion of T4 DNA with Pat I produced about 35 DNA fragments of various lengths.

At the same time, the bacterial plasmid known as pBR 325 was also cleaved with Pat I. Pat I cleaved pBR 325 provides chloramphenicol and tetracycline resistance but not ampicillin resistance. The T4 DNA fragments were then directly annealed to the Pst I cleaved pBR 325 plasmids to produce hybrid plasmids.

*E. coli* cells, strain C600 (a K12 strain) were then transformed by mixing with the pBR 325 plasmids in a dilute solution of calcium chloride. The transformed bacteria were grown first in a nonselective medium, and then in a selective medium which screened for chloroamphenicol and tetracycline resistance and ampicillin sensitivity. In this way individual transformants which had taken up the hybrid pBR 325 plasmids were isolated and cultured as colonies. Each of the colonies was given a number.

To isolate the *E. coli* transformants which contain the stabilizing DNA fragment, individual transformants were cultured. The cultures were then infected with λ0ts mutants and incubated as plate assays at 30°, 37° and 39°C. The number of plaques per plate for each culture was counted. The relative efficiency of plaque formation for selected cultures is shown in Table 1.

TABLE 1

| Plasmid | Efficiency of plaque format at: | | |
|---|---|---|---|
| | 30°C | 37°C | 39°C |
| pBR 325 | 1 | 0.38 | 0.02 |
| Pst 30 | 1 | 0.78 | 0.78 |
| Pst 83 | 1 | 1.43 | 0.76 |
| Pst 91 | 1 | 1.43 | 1.11 |

In Table 1, the designation pBR 325 indicates host cells containing the plasmid pBR 325. These cells served as a control. The designation Pst 30 indicates host cells containing the plasmid pBR 325 with the T4 DNA insert of colony 30. Similarly Pst 83 and Pst 91 indicate host cells containing the plasmid pBR 325 with the T4 DNA inserts of colonies 83 and 91 respectively.

As can be seen in Table 1, the abilty of λ0ts to propagate at 39°C was drastically reduced in *E. coli* cells having only the pBR 325 control. However, λ0ts was able to propagate well as 39°C in *E. coli* cells having the T4 DNA inserts of colonies 30, 83 and 91. The ability of λ0ts to propagate at 39°C was due to the suppressed degradation of abnormal protein in the *E. coli* cells having the T4 DNA inserts of colonies 30, 83 and 91.

Example 2

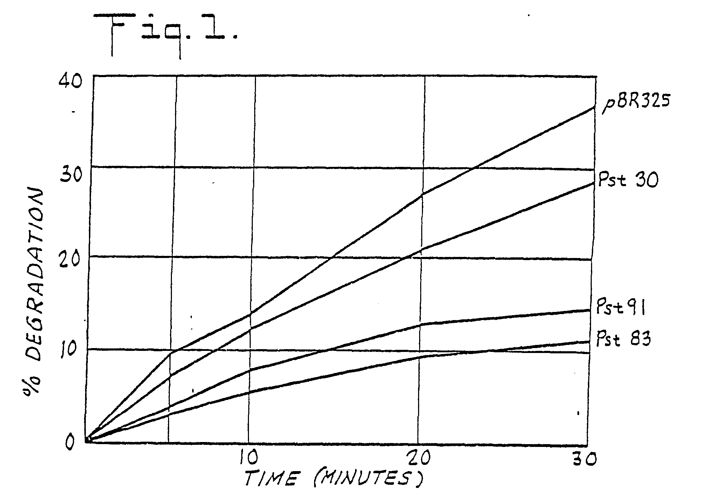

The hybrid plasmids of Example 1 were introduced into *E. coli* cells, strain SG 13062 (a K12 strain). Transformants were examined for their ability to degrade puromycyl protein fragments by the methods described in Simon et al., *Nature, supra.* The results are shown in Fig. 1, wherein the designations are the same as in Example 1.

As can be seen in Fig. 1, *E. coli* cells containing T4 DNA fragments from colonies 83 and 91 show substantially lower levels of degradation then *E. coli* cells containing only the pBR 325 plasmid. Moreover, these *E. coli* cells exhibited no change in the turnover rate of normal proteins.

Example 3

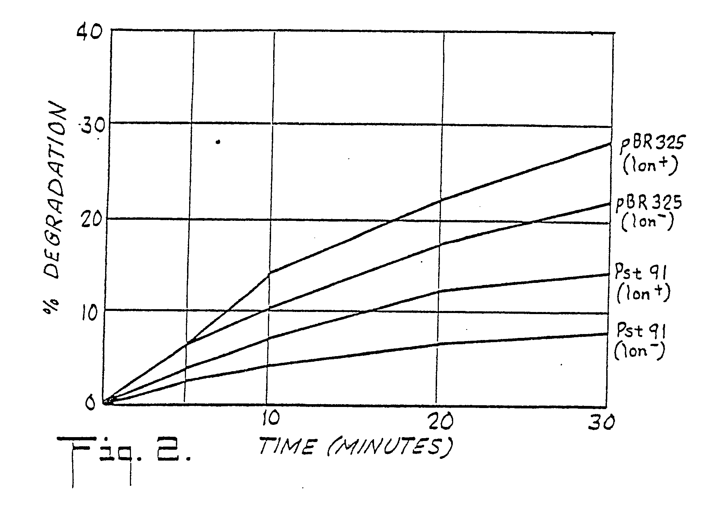

The hybrid plasmid Pst 91 was introduced into E. coli cells, strains SG 13062 and SG 13069 (both K12 strains). SG 13062 served in this example as a wild-type *E. coli* strain. SG 13069 is identical to SG 13062 except that SG 13069 carries the mutation designated *lon⁻*. This mutation of the E. coli genome reduced the efficiency of the degradation mechanism. The *E. coli* transformants were examined for their ability to

6

**0 072 925**

degrade puromycyl polypeptides. The results are shown in Fig. 2. The results demonstrate that *E. coli* cells having the stabilizing T4 DNA fragment exhibit less degradation of abnormal proteins than *E. coli lon⁻* mutants. Fig. 2 also shows that degradation is smallest in *lon⁻* mutants having the stabilizing T4 DNA fragment.

Example 4

*E. coli* cells of the strain designated 294 (a K12 strain) were transformed with plasmid Pst 91 or with pBR 325. The *E. coli* cells had previously been transformed with the plasmid designated PACY184IF. This plasmid contains the gene which codes for human fibroblast interferon. The transformants were cultured and the interferon production was assayed. The results are shown in Table 2.

TABLE 2

| Plasmids | Interferon (units/ml culture) |
|---|---|
| PACY184IF | 150 |
| PACY184IF+pBR 325 | 150 |
| PACY184IF+Pst 91 | 600 |

Table 2 demonstrates that *E. coli* clones carrying the gene for human interferon and the stabilizing T4 DNA fragment yielded four times as much interferon as clones not having the T4 DNA fragment.

Example 5

The T4 DNA fragment was excised from Pst 91 with Pst I and inserted into the plasmid designated 177. Plasmid 177 contained the gene coding for human fibroblast interferon. *E. coli* cells, strain 294, were transformed with the new plasmid designated 177—91. The transformants were cultured and the interferon was extracted after freeze-thaw cycles and assayed. The results are shown in Table 3.

TABLE 3

| Plasmid | Interferon (units/ml culture) |
|---|---|
| 177 | 150 |
| 177—91 | 600 |

As in Example 4, the *E. coli* clones carrying the gene for human interferon and the stabilizing T4 DNA fragment yielded four times as much interferon as clones not carrying the T4 DNA fragment.

Example 6

*E. coli* cells, strain 294 were transformed with the plasmid 177—91 as in Example 5. The transformants were cultured and the interferon was extracted following treatment of the cells with the detergent Triton X100. The interferon was assayed and the results are shown in Table 4.

TABLE 4

| Plasmid | Interferon (units/ml culture) |
|---|---|
| 177 | 300 |
| 177—91 | 2400 |

In this case, the host cells having the stabilizing T4 DNA fragment yielded eight times as much interferon as cells without the fragment.

While the invention has been described with reference to specific embodiments, this should not be construed to limit the spirit or the scope of the invention.

**Claims**

1. A DNA fragment consisting essentially of a sequence of nucleotide bases which code for protein that suppresses degradation of abnormal polypeptides in bacterial cells and obtainable by

treating the DNA of a T-even bacteriophage with a restriction enzyme to cleave the DNA into fragments, at least one of said fragments coding for protein that suppresses degradation in bacterial cells;

inserting said DNA fragments into cloning vehicles to produce hybrid cloning vehicles;

transforming bacterial cells by introducing said hybrid cloning vehicles therein;

selecting the transformants exhibiting suppressed degradation of abnormal proteins;

removing said hybrid cloning vehicle from a selected transformant; and

recovering the desired DNA fragment by excising it from said hybrid cloning vehicle.

2. The DNA fragment of claim 1 wherein said fragment is obtained by isolation from a T-4 bacteriophage.

3. The DNA fragment of Claim 1 wherein said T-even DNA is cytosine-containing and nonglucosylated.

4. A hybrid cloning vehicle comprising a cloning vehicle and the DNA fragment of Claim 1.

5. The hybrid cloning vehicle of Claim 4 wherein said cloning vehicle is a plasmid.

6. The hybrid cloning vehicle of Claim 4 wherein said cloning vehicle further comprises a sequence of nucleotide bases coding for a eukaryotic protein.

7. The hybrid cloning vehicle of Claim 4 wherein said cloning vehicle further comprises a sequence of nucleotide bases coding for a human protein.

8. The hybrid cloning vehicle of Claim 7 wherein said human protein is human interferon.

9. A bacterial cell containing the hybrid cloning vehicle of anyone of Claims 4 to 8.

10. A bacterial cell containing the hybrid cloning vehicle of Claim 4 and further containing a second cloning vehicle, said second cloning vehicle containing a sequence of nucleotide bases coding for a eukaryotic protein.

11. A method for suppressing the degradation of abnormal proteins in a bacterial cell, comprising producing the DNA fragment of Claim 1, inserting said DNA fragment into a cloning vehicle to produce a hybrid cloning vehicle, and transforming a bacterial cell by introducing said hybrid cloning vehicle therein.

12. A method for producing a DNA fragment consisting essentially of a sequence of nucleotide bases coding for protein that suppresses degradation of abnormal proteins in bacterial cells, comprising treating the DNA of a T-even bacteriophage with a restriction enzyme to cleave the DNA into fragments, at least one of said fragments coding for protein that suppresses degradation in bacterial cells, inserting said DNA fragments into cloning vehicles to produce hybrid cloning vehicles, transforming bacterial cells by introducing said hybrid cloning vehicles therein, and selecting the transformants exhibiting suppressed degradation of abnormal proteins.

13. The method of Claim 12 further comprising removing said hybrid cloning vehicle from a selected transformant and excising said DNA fragment from said hybrid cloning vehicle.

**Patentansprüche**

1. DNA-Fragment, das im wesentlichen aus einer Sequenz von Nucleotidbasen besteht, die für ein Protein codieren, das den Abbau von anomalen Polypeptiden in Bakterienzellen unterdrückt, und das erhältlich ist durch

Behandlung der DNA eines T-even Bacteriophagen mit einem Restriktionsenzym zur Spaltung der DNA in Fragmente, von denen zumindest eines für das Protein codiert, das den Abbau in Bakterienzellen unterdrückt;

Einfügung der DNA-Fragmente in Klonierungsvektoren zur Erzeugung von Hybrid-Klonierungsvektoren;

Transformation von Bakterienzellen durch Einführung der Hybrid-Klonierungsvektoren;

Selektion von Transformanten, die einen unterdrückten Abbau von anomalen Proteinen zeigen;

Isolierung des Hybrid-Klonierungsvektors aus einer selektionierten Transformante; und Gewinnung des gewünschten DNA-Fragments durch Herausschneiden aus dem Hybrid-Klonierungsvektor.

2. DNA-Fragment nach Anspruch 1, in welchem der Fragment durch Isolierung aus einem T-4 Bacteriophagen erhalten wird.

3. DNA-Fragment nach Anspruch 1, in welchem die T-even DNA cytosinhaltig und nicht glykosyliert ist.

4. Hybrid-Klonierungsvektor, umfassend einen Klonierungsvektor und das DNA-Fragment nach Anspruch 1.

5. Hybrid-Klonierungsvektor nach Anspruch 4, in welchem der Klonierungsvektor ein Plasmid ist.

6. Hybrid-Klonierungsvektor nach Anspruch 4, in welchem der Klonierungsvektor außerdem eine Nucleotidsequenz umfaßt, die für ein eukaryontisches Protein codiert.

7. Hybrid-Klonierungsvektor nach Anspruch 4, in welchem der Klonierungsvektor außerdem eine Nucleotidsequenz umfaßt, die für ein menschliches Protein codiert.

8. Hybrid-Klonierungsvektor nach Anspruch 7, in welchem das menschliche Protein menschliches Interferon ist.

9. Bakterienzelle, die den Hybrid-Klonierungsvektor nach einem der Ansprüche 4 bis 8 enthält.

10. Bakterienzelle, die den Hybrid-Klonierungsvektor nach Anspruch 4 und außerdem einen zweiten Klonierungsvektor enthält, wobei der zweite Klonierungsvektor eine Sequenz von Nucleotidbasen enthält, die für ein eukaryontisches Protein codieren.

11. Verfahren zur Unterdrückung des Abbaus von anomalen Proteinen in einer Bakterienzelle, umfassend die Herstellung der DNA-Fragments nach Anspruch 1, die Einfügung des DNA-Fragments in einen Klonierungsvektor zur Erzeugung eines Hybrid-Klonierungsvektors und die Transformation einer Bakterienzelle durch Einführung des Hybrid-Klonierungsvektors.

12. Verfahren zur Herstellung eines DNA-Fragments, das im wesentlichen aus einer Sequenz von Nucleotidbasen besteht, die für ein Protein codieren, das den Abbau von anomalen Proteinen in Bakterienzellen unterdrückt, umfassend die Behandlung der DNA eines T-even Bacteriophagen mit einem

**0 072 925**

Restriktionsenzym zur Spaltung der DNA in Fragmente, von denen zumindest eines für ein Protein codiert, das den Abbau in Bakterienzellen unterdrückt, die Einfügung der DNA-Fragmente in Klonierungsvektoren zur Erzeugung von Hybrid-Klonierungsvektoren, die Transformation von Bakterienzellen durch Einführung der Hybrid-Klonierungsvektoren und die Selektion von Transformanten, die einen unterdrückten Abbau von anomalen Proteinen zeigen.

13. Verfahren nach Anspruch 12, zusätzlich umfassend die Isolierung des Hybrid-Klonierungsvektors aus einer selektionierten Transformante und das Herausschneiden des DNA-Fragments aus dem Hybrid-Klonierungsvektor.

## Revendications

1. Un fragment d'ADN constitué essentiellement d'une séquence de bases nucléotidiques, qui code pour une protéine qui supprime la dégradation des polypeptides anormaux dans les cellules bactériennes, et pouvant être obtenu par

traitement de l'ADN d'un bactériophage T-pair avec une enzyme de restriction pour cliver l'ADN en fragments, au moins un desdits fragments codant pour la protéine qui supprime la dégradation dans les cellules bactériennes;

insertion desdits fragments d'ADN dans des véhicules de clonage pour produire des véhicules hybrides de clonage;

transformation de cellules bactériennes par introduction dans celles-ci desdits véhicules hybrides de clonage;

sélection des transformants présentant une suppression de la dégradation des protéines anormales;

élimination dudit véhicule hybride de clonage d'un transformant sélectionné; et

récupération du fragment d'ADN désiré par excision de ce denier dudit véhicule hybride de clonage.

2. Le fragment d'ADN de la revendication 1, ledit fragment étant obtenu par isolement à partir d'un bactériophage T4.

3. Le fragment d'ADN de la revendication 1, ledit ADN de T-pairs contenant de la cytosine et étant non glycosylé.

4. Un véhicule hybride de clonage comprenant un véhicule de clonage et le fragment d'ADN de la revendication 1.

5. Le véhicule hybride de clonage de la revendication 4, dans lequel ledit véhicule de clonage est un plasmide.

6. Le véhicule hybride de clonage de la revendication 4, dans lequel ledit véhicule de clonage comprend de plus une séquence de bases nucléotidiques codant pour une protéine d'eucaryote.

7. Le véhicule hybride de clonage de la revendication 4, dans lequel ledit véhicule de clonage comprend de plus une séquence de bases nucléotidiques codant pour une protéine humaine.

8. Le véhicule hybride de closage de la revendication 7, dans lequel ladite protéine humaine est un interféron humain.

9. Une cellule bactérienne contenant le véhicule hybride de clonage de l'une quelconque des revendications 4 à 8.

10. Une cellule bactérienne contenant le véhicule hybride de clonage de la revendication 4 et contenant de plus un second véhicule de clonage, ledit second véhicule de clonage contenant une séquence de bases nucléotidiques codant pour une protéine d'eucaryote.

11. Un procédé de suppression de la dégradation de protéines anormales dans une cellule bactérienne, comprenant la production du fragment d'ADN de la revendication 1, l'insertion dudit fragment d'ADN dans un véhicule de clonage pour produire un véhicule hybride de clonage, et la transformation d'une cellule bactérienne par introduction dudit véhicule hybride de clonage dans celle-ci.

12. Un procédé de production d'un fragment d'ADN constitué essentiellement d'une séquence de bases nucléotidiques codant pour une protéine qui supprime la dégradation des protéines anormales dans les cellules bactériennes, comprenant le traitement de l'ADN d'un bactériophage T-pair avec une enzyme de restriction pour cliver l'ADN en fragments, au moins un desdits fragments codant pour une protéine qui supprime la dégradation dans les cellules bactériennes, l'insertion desdits fragments d'ADN dans des véhicules de clonage pour produire des véhicules hybrides de clonage, la transformation de cellules bactériennes par introduction desdits véhicules hybrides de clongage dans celles-ci et la sélection des transformants présentant une suppression de la dégradation des protéines anormales.

13. Le procédé de la revendication 12, comprenant de plus l'élimination dudit véhicule hybride de clonage d'un transformant sélectionné et l'excision dudit fragment d'ADN dudit véhicule hybride de clonage.

Fig. 1.

Fig. 2.